# EUROPÄISCHE PATENTSCHRIFT

(11) **EP 2 318 409 B1**
(45) Veröffentlichungstag und Bekanntmachung des Hinweises auf die Patenterteilung: **11.07.2012**
(21) Anmeldenummer: 09777909.4
(22) Anmeldetag: 17.08.2009
(51) Int. Cl.: C07D 471/08

(54) **VERFARHEN ZUR HERSTELLUNG VON 3,7-DIAZA-BICYCLO[3.3.1]NONAN-VERBINDUNGEN**
METHOD FOR PRODUCING 3,7-DIAZA-BICYCLO[3.3.1]NONANE COMPOUNDS
PROCÉDÉ DE PRÉPARATION DE COMPOSÉS 3,7-DIAZA-BICYCLO[3.3.1]NONANES

(30) Priorität: 19.08.2008 DE 102008038376
(43) Veröffentlichungstag der Anmeldung: 11.05.2011
(73) Patentinhaber: Clariant Finance (BVI) Limited, Road Town, Tortola (VG)
(72) Erfinder: SAJITZ, Melanie, 58840 Plettenberg (DE); LABORDA, Steve, 65719 Hofheim (DE); NAUMANN, Peter, 65232 Taunusstein (DE); WESSLING, Michael, 79400 Kandern (DE)
(74) Vertreter: Mikulecky, Klaus
(86) Internationale Anmeldenummer: PCT/EP2009/005937
(87) Internationale Veröffentlichungsnummer: WO 2010/020383

(56) Entgegenhaltungen:
- WO-A1-02/48301
- WO-A1-2005/042532
- DE-A1-102005 027 619
- HALLER R: "Zur Kenntnis substituierter 3,7-Diaza- und 3-Oxa-7-aza-bicyclo-Ä3.3.1 Ü-nonanone" ARZNEIMITTEL FORSCHUNG. DRUG RESEARCH, ECV EDITIO CANTOR VERLAG, AULENDORF, DE, Bd. 15, Nr. 11, 1. Januar 1965 (1965-01-01), Seiten 1327-1330, XP002196150 ISSN: 0004-4172

## Beschreibung

Die Erfindung betrifft eine Eintopfsynthese d.h. ein verbessertes und vereinfachtes Verfahren zur Herstellung von 3,7-Diaza-bicyclo[3.3.1]nonan-Verbindungen, ohne Zwischenisolierung der Piperidone, das großtechnisch durchführbar ist, reproduzierbar gute Ausbeuten liefert und geringeren Lösemitteleinsatz erfordert.

3,7-Diaza-bicyclo[3.3.1]nonan-Verbindungen der Formel 1 stellen interessante Verbindungen für verschiedene Anwendungen dar. Unter anderem sind sie selbst oder Übergangmetallkomplexe, die Liganden gemäß Formel (I) enthalten, sehr effektive Bleichkatalysatoren 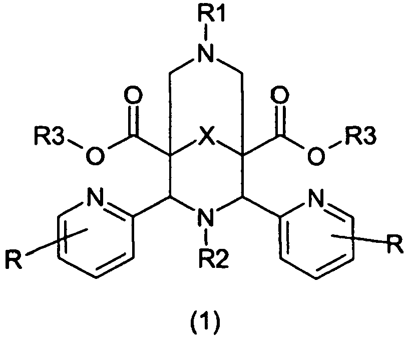 wobei R Wasserstoff, Hydroxyl, C₁-C₄ Alkyl; R¹ C₁-C₄ Alkyl, C₆-C₁₀ Aryl oder Pyridinyl-C₁-C₄-alkyl; R² C₁-C₄ Alkyl, C₆-C₁₀ Aryl; R³ C₁-C₄ Alkyl und X C=O oder C(OH)₂ bedeutet.

Deren Verwendung als Bleichkatalysator in Wasch- und Reinigungsmittel wird u. a. in WO 02/48301, US 2003/0 162 681 und WO 03/104 234 beansprucht.

Die Herstellung dieser Verbindungen wird in Inorg. Chimica Acta, 337 (2002) 407 - 419 und ebenfalls in Eur. J. Org. Chem. (2008) 1019 - 1030 im Labormaßstab als zweistufiges Verfahren beschrieben. Dies ist im großtechnischen Maßstab nicht durchführbar. Der Lösemitteleinsatz ist zu hoch und die Kristallisation erfordert eine zu lange Zeit.

Die Herstellung im großtechnischen Maßstab dieser Verbindungen erfolgt ebenfalls in zwei Stufen gemäß den Angaben in DE102005027619 (A1) nach folgendem Reaktionsschema:

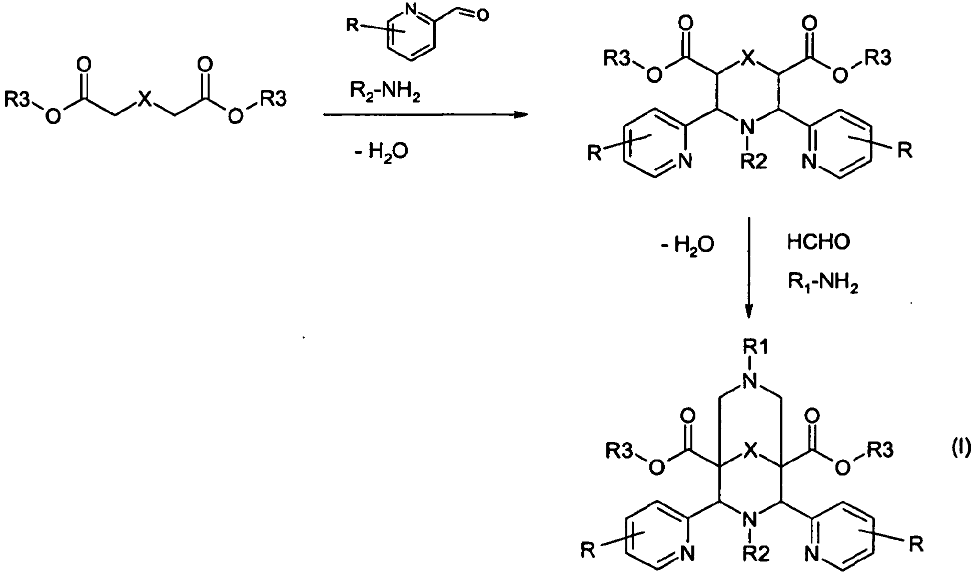

Ausgehend von Dicarbonsäurediester werden in zwei Mannich Kondensationsschritten, jeweils unter Wasserabspaltung Verbindungen gemäß Strukturformel 1 erhalten.

In einem ersten Reaktionsschritt wird bei der Synthese im großtechnischen Maßstab der Dicarbonsäurediester in einem C₁-C₄-Alkohol, wie zum Beispiel Ethanol, Propanolen oder Butanolen, bevorzugt in einem verzweigten C₃- oder C₄-Alkohol vorgelegt und auf 0 bis 20 °C abgekühlt. Zur abgekühlten Mischung wird der betreffende Pyridin-2-aldehyd getropft. Die Menge an Aldehyd beträgt 2,0 - 2,2 bevorzugt 2,0 - 2,1 Moläquivalente bezogen auf den Diester. Die Temperatur bei diesem Schritt liegt im Allgemeinen bei 0 - 20 °C, bevorzugt bei 5 - -15 °C, besonders bevorzugt bei 5 - 10 °C. Die Dosierung erfolgt innerhalb von 5 - 45 Minuten, bevorzugt innerhalb von 10 - 20 Minuten. Anschließend wird das primäre Amin R₂-NH₂ zugetropft. Die Menge an Amin beträgt 0,9 -1,1 bevorzugt 0,95 - 1,05 Moläquivalente bezogen auf den Diester. Die Temperatur bei dieser Dosierung liegt im Allgemeinen bei 0 - 20 °C, bevorzugt bei 5 - 15 °C, besonders bevorzugt bei 5 - 10 °C. Die Zugabe erfolgt über einen Zeitraum von 30 - 120 Minuten, bevorzugt innerhalb von 60 - 90 Minuten. Nach beendeter Zugabe wird die Reaktionsmischung aufgeheizt und im Vakuum durch azeotrope Destillation der Gehalt an Wasser in der Mischung verringert. Die Innentemperatur während der Destillation liegt bei 40 - 60 °C, bevorzugt bei 45 - 50 °C. Das Vakuum wird entsprechend eingestellt. Anschließend wird abgekühlt und nachgerührt, wobei die Temperatur bei 0 - 20 °C liegt. Nach beendeter Nachrührzeit wird das Produkt abfiltriert, mit Lösemittel gewaschen und getrocknet.

Nach dem zweistufigen Verfahren wird das Zwischenprodukt in Ausbeuten > 80 %, bevorzugt in Ausbeuten von 84 - 88 % und hoher Reinheit (> 95 % Gehalt nach NMR) erhalten.

In einem zweiten Reaktionsschritt wird das Produkt der ersten Stufe wiederum in einem C₃-C₄-Alkohol, wie zum Beispiel in Propanolen oder Butanolen, bevorzugt verzweigten C₃- oder C₄-Alkoholen suspendiert. Der Alkohol in diesem Reaktionsschritt ist vorzugsweise der gleiche Alkohol wie im ersten Reaktionsschritt. In beiden Reaktionsschritten können jedoch auch unterschiedliche Alkohole innerhalb der gegebenen Definition genommen werden. Nacheinander werden das Amin R¹-NH₂ und Formalinlösung zugegeben. Die Menge an Amin beträgt 1,2 - 1,6 bevorzugt 1,4 - 1,5 Moläquivalente bezogen auf das Zwischenprodukt, die Menge an Formaldehyd beträgt 3,0 - 4,5 Moläquivalente bezogen auf das Produkt der ersten Stufe. Anschließend wird die Mischung erwärmt und nachgerührt. Die Reaktionszeit beträgt 1 - 3 Stunden, bevorzugt, 1,5 - 2 Stunden, die Temperatur liegt bei 50 - 70 °C, bevorzugt bei 55 - 65 °C. Anschließend wird unter Vakuum durch azeotrope Destillation der Gehalt an Wasser im Reaktionsgemisch so weit wie möglich verringert. Nach beendeter Destillation wird zunächst Raumtemperatur dann auf 0 - 15 °C bevorzugt auf 5 - 10 °C abgekühlt und nachgerührt. Dann wird das Produkt abfiltriert, mit frischem Lösemittel gewaschen und getrocknet.

Nach dem zweistufigen Verfahren wird die zweite Stufe der Formel I in Ausbeuten > 50 % bevorzugt in Ausbeuten von 55 - 56 % in Reinheiten > 98 % (Gehalt nach NMR) erhalten.
Die Ausbeute über zwei Stufen beträgt 44 bis 56 %.

Das beschriebene Syntheseverfahren erfordert für die Isolierung des Zwischenproduktes hohe Lösemittelzusätze zur Reinigung und sehr lange Trocknungszeiten, die Isolierung der festen Produkte aus den Reaktionsapparaturen ist mit einem sehr hohen Aufwand verbunden und ist so technisch schwierig durchführbar. Die schlechte Rieselfähigkeit des Zwischenproduktes ist der Grund für eine schwierige Handhabung. Die Isolation des Zwischenproduktes führt ebenfalls zu einer drastischen Verteuerung des Endproduktes.

Aufgabe war deshalb, ein verbessertes und kostengünstigeres Verfahren zur Herstellung von Substanzen der Formel I zu entwickeln, das frei ist von den oben geschilderten Nachteilen. Es wurde nun gefunden, dass die in Rede stehenden Verbindungen auch durch ein Einstufen-Verfahren hergestellt werden können.

Gegenstand der Erfindung ist ein Verfahren zur Herstellung von 3,7-Diaza-bicyclo[3.3.1]nonan-Verbindungen der Formel 1 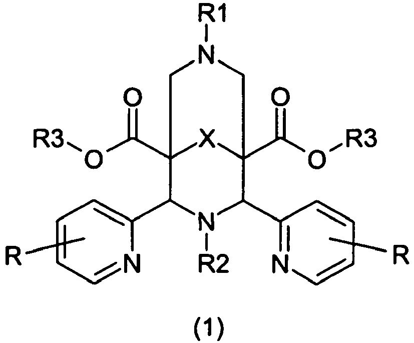 wobei R Wasserstoff, Hydroxyl, C₁-C₄ Alkyl; R¹ C₁-C₄ Alkyl, C₆-C₁₀ Aryl oder Pyridinyl-C₁-C₄-alkyl; R² C₁-C₄ Alkyl, C₆-C₁₀ Aryl; R³ C₁-C₄ Alkyl und X C=O oder C(OH)₂ bedeutet, durch
(a) Umsetzung eines Dicarbonsäureesters der Formel (2) 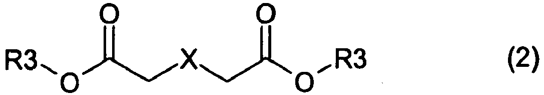 mit einem Pyridinaldehyd der Formel (3) 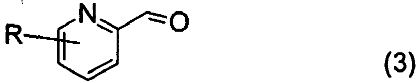 sowie einem Amin der Formel (4)

   R²-NH₂ (4)

   zu einem Piperidon der Formel (5) 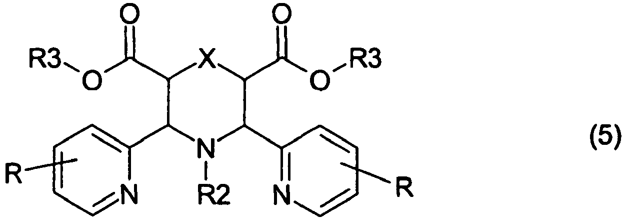 und
(b) Umsetzung der so erhaltenen Piperidone der Formel (5) mit Formaldehyd und einem Amin der Formel R¹-NH₂, wobei R, R¹, R² und R³ die oben genannten Bedeutungen haben. Dieses Verfahren besteht darin, dass man die beiden Reaktionsstufen in einem C₁-C₄-Alkohol durchführt ohne Zwischenisolierung der Piperidone der Formel (5) und jeweils das entstandene Reaktionswasser durch azeotrope Destillation entfernt.

Das erfindungsgemäße Verfahren im Einzelnen erfolgt in folgender Weise:
Der Dicarbonsäurediester wird in einem C₁-C₄-Alkohol, wie zum Beispiel in Methanol, Ethanol, Propanolen oder Butanolen, bevorzugt in einem verzweigten C₃- oder C₄-Alkohol vorgelegt und auf 0 - 20 °C abgekühlt. Zur abgekühlten Mischung wird der betreffende Pyridin-2-aldehyd zugetropft. Die Menge an Aldehyd beträgt 2,0 - 2,2 bevorzugt 2,0 - 2,1 Moläquivalente bezogen auf den Diester. Die Temperatur bei diesem Schritt liegt im Allgemeinen bei 0 - 20 °C, bevorzugt bei 5 - 15 °C, besonders bevorzugt bei 5 - 10 °C. Die Dosierung erfolgt innerhalb von 5 - 45 Minuten, bevorzugt innerhalb von 10 - 20 Minuten. Anschließend wird das primäre Amin R₂-NH₂ zugetropft. Die Menge an Amin beträgt 0,9 - 1,1 bevorzugt 0,95 - 1,05 Moläquivalente bezogen auf den Diester. Die Temperatur bei dieser Dosierung liegt im Allgemeinen bei 0 - 20 °C, bevorzugt bei 5 - -15 °C, besonders bevorzugt bei 5 - 10 °C. Die Zugabe erfolgt über einen Zeitraum von 30 - 120 Minuten, bevorzugt innerhalb von 60 - 90 Minuten. Nach beendeter Zugabe wird die Reaktionsmischung aufgeheizt und im Vakuum durch azeotrope Destillation der Gehalt an Wasser in der Mischung verringert. Hierbei werden mindestens 80 % des Reaktionswassers entfernt. Gleichzeitig wird ein C₁-C₄-Alkohol, wie zum Beispiel Methanol, Ethanol, Propanole oder Butanole, bevorzugt ein verzweigter C₃- oder C₄-Alkohol zudosiert. Der Alkohol in diesem Reaktionsschritt ist vorzugsweise derselbe Alkohol wie beim Vorlegen und beträgt die 0,8 fache Menge davon. Die Innentemperatur während der Destillation liegt bei 40 - 60 °C, bevorzugt bei 45 - 50 °C. Das Vakuum wird entsprechend eingestellt. Anschließend wird abgekühlt und nachgerührt, wobei die Temperatur bei 0 - 20 °C liegt. Nacheinander werden dann das Amin R¹-NH₂ und Formalinlösung zugegeben. Die Menge an Amin beträgt 1,2 - 1,6 bevorzugt 1,4 - 1,5 Moläquivalente bezogen auf das Zwischenprodukt Piperidon der Formel (5) (theoretischer Umsatz von 90 %), die Menge an Formaldehyd beträgt 2,8 - 4,5 bevorzugt 3,0 Moläquivalente bezogen auf das Zwischenprodukt der Formel (5). Anschließend wird die Mischung erwärmt und nachgerührt. Die Reaktionszeit beträgt 1 - 3 Stunden, bevorzugt, 1,5 - 2 Stunden, die Temperatur liegt bei 50 - 70 °C, bevorzugt bei 55 - 65 °C. Anschließend wird unter Vakuum durch azeotrope Destillation der Gehalt an Wasser im Reaktionsgemisch so weit wie möglich verringert. Hierbei werden mindestens 80 % des Reaktionswassers entfernt. Gleichzeitig wird ein frischer C₁-C₄-Alkohol, wie zum Beispiel Methanol, Ethanol, Propanole oder Butanole, bevorzugt in einem verzweigten C₃- oder C₄-Alkohol zugeführt. Der Alkohol in diesem Reaktionsschritt ist vorzugsweise derselbe Alkohol wie beim Vorlegen und beträgt die 2,4 fache Menge davon. Nach beendeter Destillation wird zunächst innerhalb von 60 Minuten auf Raumtemperatur abgekühlt und 0,5 - 12 Stunden nachgerührt. Dann wird das Produkt abfiltriert, mit Lösemittel (ein frischer C₁-C₄-Alkohol, wie zum Beispiel Methanol, Ethanol, Propanole oder Butanole) gewaschen und getrocknet.

Nach dem erfindungsgemäßen Verfahren wird die Verbindung der Formel I in Ausbeuten > 70 % bevorzugt in Ausbeuten von 75 - 80 % in hoher Reinheit erhalten (> 98 % Gehalt nach NMR).

Die Herstellung der Verbindungen gemäß Formel 1 nach dem erfindungsgemäßen Verfahren erfordert im Vergleich zum Stand der Technik
- ein Eintopfverfahren mit besserer Ausbeute anstatt zweier Reaktionsschritte mit aufwendiger und problematischer Isolierung der ersten Stufe
- eine erheblich reduzierte Masse an benötigtem organischem Lösemittel. Das Verfahren ist im Vergleich zum Stand der Technik in einem Schritt ohne Isolierung des Zwischenproduktes und daher vereinfacht großtechnisch durchführbar und führt zu einer besseren Ausbeute, d. h. es ist erheblich kostengünstiger.

Das Verfahren, wie in der Literatur beschrieben, ist großtechnisch in vielen Punkten nicht realisierbar, die Isolierung der Zwischenstufe der Formel (5) ist aufwendig und dementsprechend zu teuer. Durch Einführung des Eintopfverfahrens konnte diese Problematik gelöst werden. Das Eintopfverfahren garantiert eine effektivere Durchführbarkeit mit verbesserter Ausbeute.

Nachfolgendes Beispiel soll die Erfindung näher erläutern ohne sie darauf einzuschränken.

### Beispiel

11,2 kg Acetondicarbonsäuredimethylester (97 %ig; 64 mol) wurden in 15 kg (19 l) iso-Butanol gelöst. Die Lösung wurde auf 10 °C abgekühlt. Bei dieser Temperatur wurden 13,4 kg Pyridin-2-aldehyd (99 %ig, 125 mol) in 10 kg (13 l) iso-Butanol zugetropft und 10 Minuten nachgerührt. Zu dieser Mischung wurden anschließend 4,8 kg Methylamin (40 % in Wasser, 62 mol) innerhalb von zwei Stunden so zugetropft, dass die Temperatur bei gleich bleibender Kühlung gehalten werden konnte. Die Reaktionsmischung wurde innerhalb von 30 Minuten auf Raumtemperatur und dann innerhalb von 30 Minuten auf 40 - 45 °C erwärmt. Danach wurde im Vakuum (anfänglich 200 - 150 mbar, dann 40 - 50 mbar) bei 40 - 45 °C Innentemperatur im Azeotrop 16 kg (17 l) iso-Butanol und Wasser abdestilliert. Währenddessen wurden 12 kg (15 l) iso-Butanol kontinuierlich zudosiert. Im Anschluss wurde mit Stickstoff belüftet und auf Raumtemperatur abgekühlt.
Zum Reaktionsgemisch wurden 8,4 kg Aminomethylpyridin (78 mol, bezogen auf das Zwischenprodukt der Formel (5) in theoretischer Ausbeute von 90 %) zudosiert und der Dosiertrichter mit 7,0 kg (9 l) iso-Butanol nachgewaschen. Dann wurden 13,5 kg Formaldehydlösung (37 % in Wasser, 166,5 mol, bezogen auf das Zwischenprodukt) innerhalb von 15 - 30 Minuten zugegeben. Nach beendeter Zugabe wurde die Mischung innerhalb von 30 Minuten auf 55 - 60 °C erwärmt und 1,5 Stunden bei dieser Temperatur gerührt. Anschließend wurden bei maximal 60 °C Innentemperatur bei zunächst 200 - 150 mbar dann 100 - 90 mbar 55 kg (60 l) als azeotropes Gemisch iso-Butanol und Wasser abdestilliert. Während der Destillation wurde 36 kg (45 l) iso-Butanol kontinuierlich zudosiert. Es wurde mit Stickstoff belüftet und innerhalb einer Stunde auf Raumtemperatur abgekühlt. Nach 12 h Rühren bei 25 °C wurde der Niederschlag abfiltriert, mit jeweils dreimal 10 kg (13 1) iso-Butanol gewaschen und im Vakuum bei 50 °C getrocknet. Es wurden 23,3 kg (72,1 %) 2, 4-Di-(pyridyl)-3-methyl-7-(pyridin-2-ylmethyl)-3, 7-diazabicyclo[3.3.1]nonan-9-on-1, 5-dimethyl-dicarboxylat in Form eines farblosen Pulvers erhalten.

## Patentansprüche

1. Verfahren zur Herstellung von 3,7-Diaza-bicyclo[3.3.1]nonan-Verbindungen der Formel 1 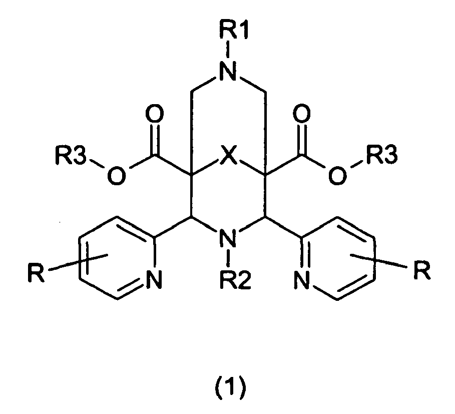 wobei R Wasserstoff, Hydroxyl, C₁-C₄ Alkyl; R¹ C₁-C₄ Alkyl, C₆-C₁₀ Aryl, Pyridinyl-C₁-C₄-alkyl; R² C₁-C₄ Alkyl, C₆-C₁₀ Aryl; R³ C₁-C₄ Alkyl und X C=O oder C(OH)₂ bedeutet, durch
(a) Umsetzung eines Dicarbonsäureesters der Formel (2) 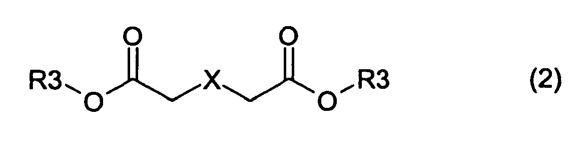 mit einem Pyridinaldehyd der Formel (3) 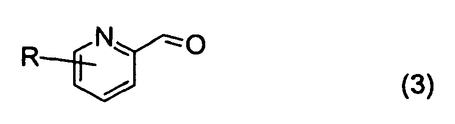
sowie einem Amin der Formel (4)
R²-NH₂ (4)
zu einem Piperidon der Formel (5) 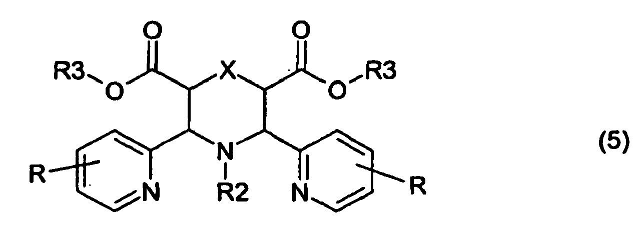 und
(b) Umsetzung der so erhaltenen Piperidone der Formel (5) mit Formaldehyd und einem Amin der Formel R¹-NH₂, wobei R, R¹, R² und R³ die oben genannten Bedeutungen haben, **dadurch gekennzeichnet, dass** man die Reaktionsstufen (a) und (b) in einem C₁-C₄-Alkohol durchführt und das jeweils entstandene Reaktionswasser durch azeotrope Destillation entfernt, wobei gleichzeitig ein C₁-C₄-Alkohol zudosiert wird, ebenfalls **dadurch gekennzeichnet, dass** man die Reaktionsstufen (a) und (b) in einem Eintopfverfahren ohne Isolierung des Zwischenproduktes (5) ausführt.

2. Verfahren nach Anspruch 1, **dadurch gekennzeichnet, dass** man die Reaktionsstufen (a) und (b) in einem verzweigten C₃- oder C₄-Alkohol durchführt.

3. Verfahren nach Anspruch 1, **dadurch gekennzeichnet, dass** man die Reaktionsstufen (a) und (b) in iso-Butanol durchführt.

## Claims

1. A method for producing 3,7-diazabicyclo[3.3.1]nonane compounds of the formula 1 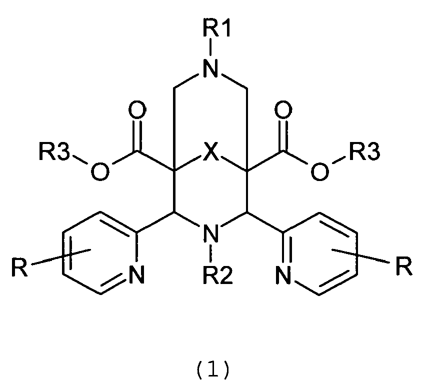 where R is hydrogen, hydroxyl, C₁-C₄-alkyl; R¹ is C₁-C₄-alkyl, C₆-C₁₀-aryl, pyridinyl-C₁-C₄-alkyl; R² is C₁-C₄-alkyl, C₆-C₁₀-aryl; R³ is C₁-C₄-alkyl and X is C=O or C(OH)₂, by
(a) reacting a dicarboxylic acid ester of the formula (2) 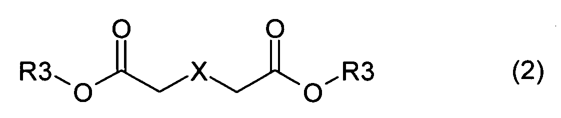 with a pyridinealdehyde of the formula (3) 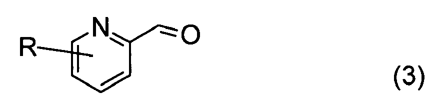 and also an amine of the formula (4)
R²-NH₂ (4)
to give a piperidone of the formula (5) 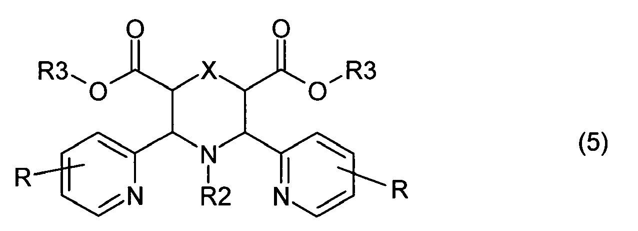 and
(b) reacting the piperidones of the formula (5) obtained in this way with formaldehyde and an amine of the formula R¹-NH₂, where R, R¹, R² and R³ have the meanings given above, wherein the reaction stages (a) and (b) are carried out in a C₁-C₄-alcohol and the water of reaction that is formed in each case is removed by azeotropic distillation, where, at the same time, a C₁-C₄-alcohol is metered in, likewise wherein the reaction stages (a) and (b) are carried out in a one-pot method without isolation of the intermediate product (5).

2. The method as claimed in claim 1, wherein the reaction stages (a) and (b) are carried out in a branched C₃- or C₄-alcohol.

3. The method as claimed in claim 1, wherein the reaction stages (a) and (b) are carried out in isobutanol.

## Revendications

1. Procédé de fabrication de composés de 3,7-diaza-bicyclo[3.3.1]nonane de formule 1 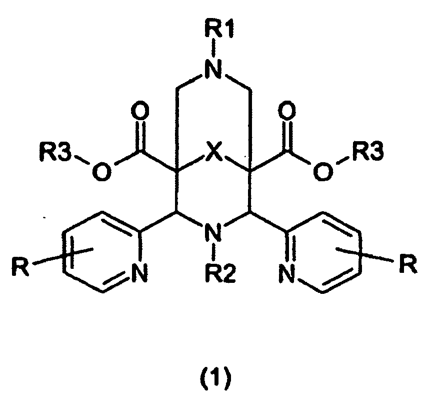 dans laquelle R signifie hydrogène, hydroxyle, alkyle en C₁-C₄ ; R¹ signifie alkyle en C₁-C₄, aryle en C₆-C₁₀, pyridinyl-alkyle en C₁-C₄ ; R² signifie alkyle en C₁-C₄, aryle en C₆-C₁₀ ; R³ signifie alkyle en C₁-C₄ et X signifie C=O ou C(OH)₂, par
(a) la mise en réaction d'un ester d'acide dicarboxylique de formule (2) 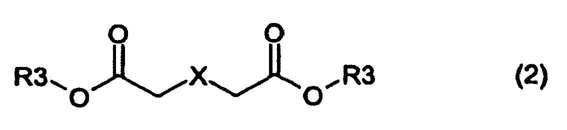
avec un pyridine-aldéhyde de formule (3) 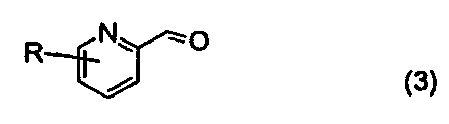 ainsi qu'avec une amine de formule (4)
R²-NH₂ (4)
pour former une pipéridone de formule (5) 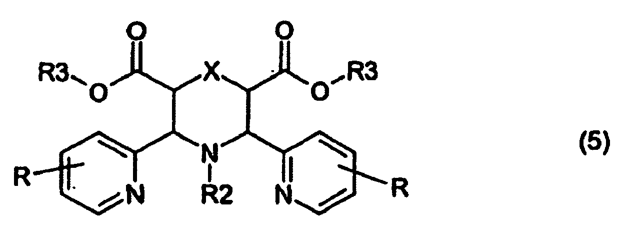 et
(b) la mise en réaction des pipéridones de formule (5) ainsi obtenues avec du formaldéhyde et une amine de formule R¹-NH₂, R, R¹, R² et R³ ayant les significations susmentionnées, **caractérisé en ce que** les étapes de réaction (a) et (b) sont réalisées dans un alcool en C₁-C₄ et l'eau de réaction formée à chaque fois est éliminée par distillation azéotropique, un alcool en C₁-C₄ étant ajouté simultanément, également **caractérisé en ce que** les étapes de réaction (a) et (b) sont réalisées dans un procédé monotope sans isolement du produit intermédiaire (5).

2. Procédé selon la revendication 1, **caractérisé en ce que** les étapes de réaction (a) et (b) sont réalisées dans un alcool en C₃ ou C₄ ramifié.

3. Procédé selon la revendication 1, **caractérisé en ce que** les étapes de réaction (a) et (b) sont réalisées dans de l'iso-butanol.
